# EUROPÄISCHE PATENTSCHRIFT

(11) **EP 0 545 154 B1**
(45) Veröffentlichungstag und Bekanntmachung des Hinweises auf die Patenterteilung: **14.09.1994**
(21) Anmeldenummer: 92119694.5
(22) Anmeldetag: 19.11.1992
(51) Int. Cl.: C07D 301/00, C07D 303/26, C08G 59/24

(54) **Verfahren zur Herstellung von cycloalifatischen Diglycidyloligoethern durch katalytische Hydrierung der entsprechenden aromatischen Ether**
Process for the preparation of diglycidyl oligoethers by catalytic hydrogenation of the corresponding aromatic ethers
Procédé pour la préparation de diglycidyl oligoéthers par hydrogénation catalytique des éthers aromatiques correspondants

(30) Priorität: 29.11.1991 DE 4139255
(43) Veröffentlichungstag der Anmeldung: 09.06.1993
(73) Patentinhaber: BASF Aktiengesellschaft, 67063 Ludwigshafen (DE)
(72) Erfinder: Schuster, Ludwig, Dr., W-6703 Limburgerhof (DE); Herzog, Rolf, Dr., W-4250 Bottrop (DE)

(56) Entgegenhaltungen:
- EP-A- 0 258 789
- EP-A- 0 402 743
- FR-A- 2 134 619
- US-A- 3 336 241
- US-A- 3 799 950
- H. JAHN 'Epoxidharze' 1969 , VEB DEUTSCHER VERLAG FÜR GRUNDSTOFFINDUSTRIE , LEIPZIG
- B. VOLLMERT, Grundriss der Makromolekularen Chemie, Bd. II, s. 246.
- Encyclopedia of Polymer Science, 2nd Ed., vol. 14, New York 1988, s. 101-169

## Beschreibung

Die vorliegende Erfindung betrifft ein neues Verfahren zur Herstellung von Diglycidyloligoethern der Formel I 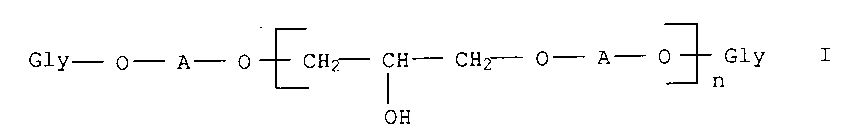
in der Gly den Glycidylrest 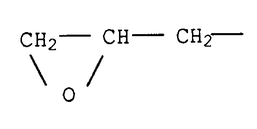
und A die Gruppierung 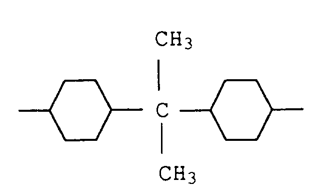
bedeuten und n einen mittleren Wert von 5 bis 10 hat, durch katalytische Hydrierung eines Diglycidyloligoethers der Formel II 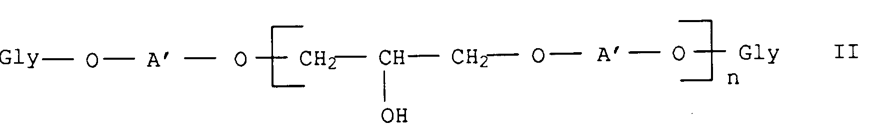
in der A' für die Gruppierung 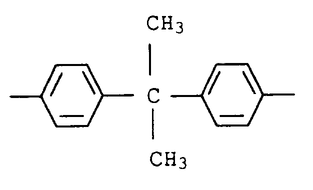
steht, mittels eines Edelmetallkatalysators.

Die US-A 4 303 579 betrifft Umsetzungsprodukte aus ungesättigten Monocarbonsäuren und Diglycidyloligoethern, darunter besonders solchen der Formel I. Nach den dortigen Ausführungen sind diese Verbindungen I entweder durch Umsetzung von Epichlorhydrin mit 2,2-Di-(4-hydroxy-cyclohexyl)propan oder durch katalytische Kernhydrierung der entsprechenden aromatischen Diglycidyloligoether II erhältlich (vgl. Spalte 3, Zeilen 20 bis 55).

Bezüglich der letztgenannten Methode wird auf die US-A 3 336 241 verwiesen, derzufolge man u.a. aromatische Epoxyverbindungen durch Kernhydrierung mit Hilfe von Rhodium- oder Ruthenium-Trägerkatalysatoren hydrieren könne. Ausführlich erläutert wird dieses Verfahren für die Hydrierung des 2,2-Bis-(4-hydroxyphenyl)-propans ("Bisphenol A") unter Verwendung von Rhodium-Trägerkatalysatoren, wobei allerdings mit steigendem Kernhydrierungsgrad auch die Epoxigruppen hydrogenolytisch gespalten werden, und im Falle eines Oligoethers vom Typ I (n = rd. 3) werden 7 % der Epoxigruppen zerstört, wenn der Hydrierungsgrad 85 % beträgt (Beispiel XII). Gänzlich unbefriedigend gelingt die Hydrierung des Diglycidylethers des Bisphenols A mit einem Ruthenium-Trägerkatalysator unter einem Wasserstoffdruck von rd. 7 bar, denn in diesem Falle beträgt der Verlust an Epoxygruppen 39 %, wobei der Kernhydrierungsgrad unter 35 % liegt.

Aus der EP-A 258 789 ist ferner die Kernhydrierung des Diglycidylethers von Bisphenol A mittels Rutheniumkatalysatoren bei einem Wasserstoffdruck über 100 bar bekannt, wobei nach dem dortigen Verfahrensbeispiel Rutheniumoxidhydrat als Katalysator verwendet wurde.

Die EP-A-402 743 lehrt die Kernhydrierung von Di-[p-glycidoxi-phenyl]-methan mittels eines Rutheniumkatalysators bei 30 bis 60°C und Drücken oberhalb von 100 bar.

Der Erfindung lag nun die Aufgabe zugrunde, die Diglycidyloligoether I besser zugänglich zu machen, und zwar mit dem speziellen Ziel, daß I möglichst keine aromatischen Einheiten oder olefinische Doppelbindungen enthält und daß die beiden endständigen Epoxidgruppen zumindest weitgehend intakt bleiben. Wird insbesondere die letztgenannte Bedingung nicht erfüllt, erhält man neben den Bisepoxiverbindungen I monofunktionelle Epoxide, welche als Polymerbausteine höchst unerwünscht sind, weil sie zu Kettenabbrüchen führen und keine Vernetzung mehr bewirken können.

Dieser Aufgabe entsprechend wurde ein Verfahren zur Herstellung der eingangs definierten Verbindungen I aus den aromatischen Verbindungen II durch katalytische Hydrierung mittels eines Edelmetallkatalysators gefunden, welches dadurch gekennzeichnet ist, daß man hierzu Rutheniumoxidhydrat als Katalysator verwendet und die Hydrierung bei 20 bis 60°C und bei einem wasserstoffdruck von 100 bis 400 bar vornimmt.

Die Ausgangsverbindungen II sind bekannt oder in bekannter Weise durch Umsetzung von Bisphenol A mit Epichlorhydrin in Gegenwart von Alkalien erhältlich (s. z.B. Houben-Weyl, Methoden der Organischen Chemie, E 20, 1987, S. 1916-1924). Die Einstellung des gewünschten Kondensationsgrades n erfolgt hierbei in bekannter Weise durch Wahl des Molverhältnisses vom Epichlorhydrin zum Bisphenol A.

Das als Hydrierkatalysator dienende Rutheniumoxidhydrat entspricht der Formel Ru₂O₃ . x H₂O, wobei x unterschiedliche Werte über 1 annehmen kann. Man erhält es als wasserfeuchten Niederschlag durch Umsetzung einer wäßrigen Lösung von Ruthenium-III-chloridhydrat RuCl₃ . 3 H₂O mit Natronlauge und anschließendes Waschen mit Wasser zur Entfernung der Chloridionen. Man verwendet es in wäßriger Suspension oder vorzugsweise als Suspension in einem Lösungsmittel, in welchem sich I und II lösen.

Die Menge des Katalysators ist nicht kritisch, sondern hat lediglich einen Einfluß auf die Hydriergeschwindigkeit. Zur Erzielung befriedigender Raum-Zeit-Ausbeuten verwendet man zweckmäßigerweise 1 bis 10 g Ruthenium in Form des Oxidhydrats pro kg der Ausgangsverbindung II.

Die Hydrierung wird bei Drücken oberhalb von 100 bar, bevorzugt im Bereich von 200 bis 325 bar durchgeführt; höhere Drücke als 400 bar bringen keine erkennbaren Vorteile mehr.

Die Reaktionstemperatur liegt zwischen 20 und 60°C, vorzugsweise zwischen 40 und 50°C. Erhöht man die Temperatur über 60°C, tritt verstärkt Hydrierung der Epoxidgruppen ein. Bei tieferen Temperaturen als 20°C werden die Aromaten nicht vollständig hydriert.

Es hat sich als vorteilhaft erwiesen, die Hydrierung in Lösung durchzuführen. Als Lösungsmittel bevorzugt werden Ether wie Glykoldimethylether und Methyl-tert.-butylether, besonders bevorzugt werden Dioxan und Tetrahydrofuran.

Die Reaktionszeiten liegen zwischen 5 und 30 Stunden.

Die Diglycidyloligoether I sind wertvolle Bausteine für die Herstellung von Kunststoffen, darunter besonders von Epoxiharzen. Sie zeichnen sich gegenüber den aromatischen Vorstufen II durch eine niedrigere Viskosität aus.

### Beispiel

Eine Lösung aus 1,2 kg eines Diglycidyloligoethers II, dessen mittlerer Kondensationsgrad einem Wert für n = 7,1 entsprach, und 1,1 1 Tetrahydrofuran wurden mit 120 ml einer Suspension von Rutheniumoxidhydrat in Tetrahydrofuran versetzt, deren Rutheniummenge 3 g betrug. Dieses Gemisch wurde für die Dauer von 20 Stunden der Hydrierung bei 50°C unter einem Wasserstoffdruck von 200 bar unterworfen. Die übliche Aufarbeitung lieferte das hydrierte Produkt I in Form einer farblosen glasartigen Substanz mit einem Kernhydrierungsgrad von rd. 95 %. Von den Epoxigruppen blieben über 95 % intakt.

## Patentansprüche

1. Verfahren zur Herstellung von Diglycidyloligoethern der Formel I 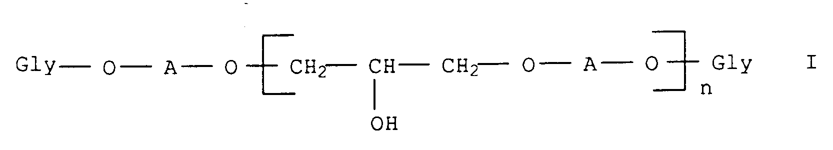 in der Gly den Glycidylrest 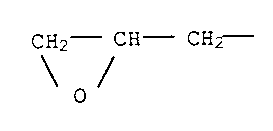 und A die Gruppierung 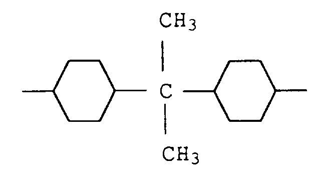 bedeuten und n einen mittleren Wert von 5 bis 10 hat, durch katalytische Hydrierung eines Diglycidyloligoethers der Formel II 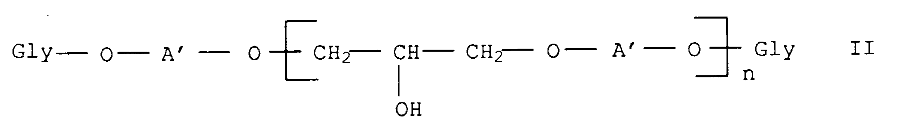 in der A' für die Gruppierung 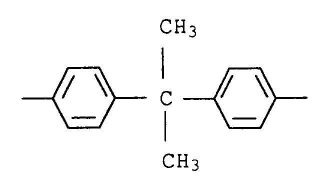 steht, mittels eines Edelmetallkatalysators, dadurch gekennzeichnet, daß man hierzu Rutheniumoxidhydrat als Katalysator verwendet und die Hydrierung bei 20 bis 60°C und einem Wasserstoffdruck von 100 bis 400 bar vornimmt.

## Claims

1. A process for the preparation of diglycidyl oligoethers of the formula I 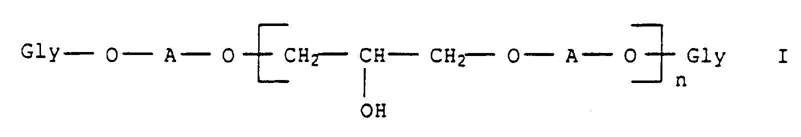 where Gly is the glycidyl radical 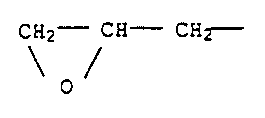 and A is the group 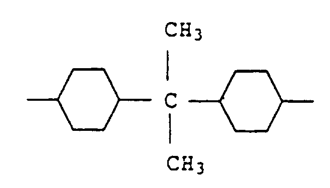 and n has an average value of from 5 to 10, by catalytic hydrogenation of a diglycidyl oligoether of the formula II 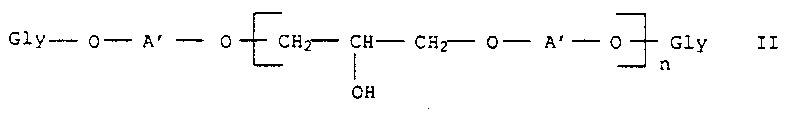 where A' is the group 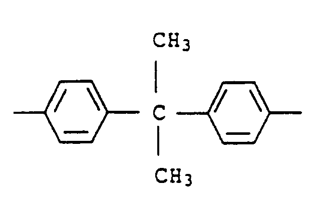 by means of a noble metal catalyst, wherein ruthenium oxide hydrate is used as catalyst for this, and the hydrogenation is carried out at 20 to 60°C and under a pressure of from 100 to 400 bar of hydrogen.

## Revendications

1. Procédé de préparation d'oligoéthers diglycidyliques de la formule I 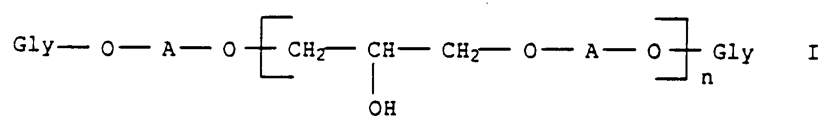 dans laquelle Gly représente le reste glycidyle de la formule 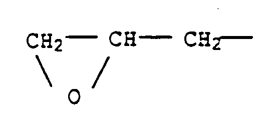 et A représente le groupement 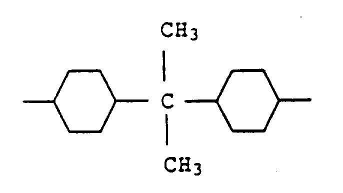 et n possède une valeur moyenne de 5 à 10, par l'hydrogénation catalytique d'un oligoéther diglycidylique de la formule II 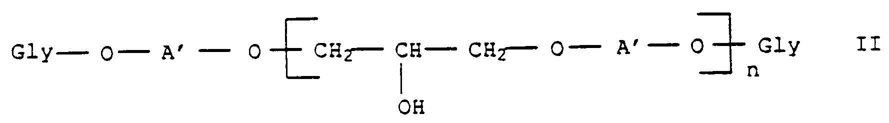 dans laquelle A' représente le groupement de la formule 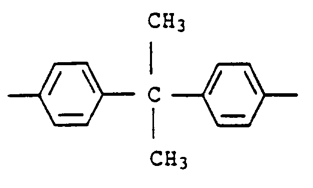 à l'aide d'un catalyseur à métal noble, caractérisé en ce que l'on utilise de l'oxyde de ruthénium hydraté à titre de catalyseur et on entreprend l'hydrogénation à une température de 20 à 60°C et sous une pression d'hydrogène de 100 à 400 bars.
